(19) Europäisches Patentamt
European Patent Office
Office européen des brevets

(11) Publication number: 0 163 526
A1

# (12) EUROPEAN PATENT APPLICATION

(21) Application number: 85303771.1

(22) Date of filing: 29.05.85

(51) Int. Cl.⁴: C 07 C 133/02

(30) Priority: 31.05.84 US 615623

(43) Date of publication of application: 04.12.85 Bulletin 85/49

(84) Designated Contracting States: BE DE FR GB IT NL

(71) Applicant: OLIN CORPORATION
350 Knotter Drive
Cheshire Connecticut 06410-0586(US)

(72) Inventor: Davidson, Eugene Passarge
Colebrook Stage
Winsted Connecticut 06098(US)

(72) Inventor: Gavin, David Francis
255 Sorghum Mill Drive
Cheshire Connecticut 06410(US)

(72) Inventor: Schiessl, Henry William
79 Parsonage Hill Road
Northford Connecticut 06472(US)

(74) Representative: Thomas, Roger Tamlyn et al,
D. Young & Co. 10 Staple Inn
London WC1V 7RD(GB)

## (54) Process for preparing hydrazodicarbonamide.

(57) An improved process for preparing hydrazodicarbonamide comprising:

(a) reacting urea with hydrazine in a solvent at a temperature of from about 105°C to about 140°C and at a pressure of at least about 15 psig for a sufficient amount of time to form an aqueous reaction mixture which is substantially free of hydrazine and comprises water, unreacted urea, semicarbazide, and ammonia; and

(b) then converting said reaction mixture into hydrazodicarbonamide;

wherein the improvement comprises

(c) passing an inert gas through said reaction mixture during step (b) to sweep out ammonia from said reaction mixture;

(d) removing substantially said ammonia from said inert gas; and

(e) recycling said ammonia-depleted inert gas back to said reaction mixture.

EP 0 163 526 A1

C-9030



# PROCESS FOR PREPARING HYDRAZODICARBONAMIDE

## Background of the Invention

### 1. Field of the Invention

This invention relates to a process for preparing hydrazodicarbonamide.

### 2. Brief Summary of the Invention

Hydrazodicarbonamide is the chemical intermediate for azodicarbonamide, a commercial blowing agent used in the manufacture of foams and other cellular materials.

Hydrazocarbonamide has heretofore been prepared by (1) reacting carbon monoxide with hydrazine in the presence of a metal carbonyl catalyst (see U.S. Patent Reissue 24,526); (2) reacting urea with hydrazine, or its salts (see U.S. Patent Nos. 2,692,281; 3,227,753 and 3,505,308); (3) reacting urea with a ketazine with or without the presence of an acid (see U.S. Patent Nos. 3,969,446; 4,049,712; and 4,176,135).

The formation of hydrazodicarbonamide by reacting urea with hydrazine is the only commercially important process today. The stoichiometry of this reaction is shown by Equation (A) as follows:

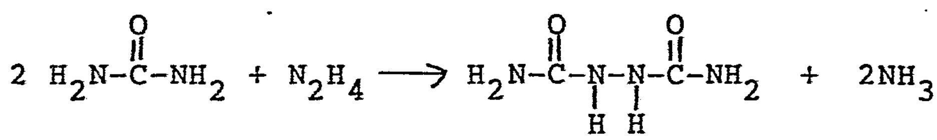 (A)

It should be noted that this reaction occurs in two stages through the intermediate, semicarbazide. The following Equation (B) illustrates the first stage, the formation of semicarbazide.

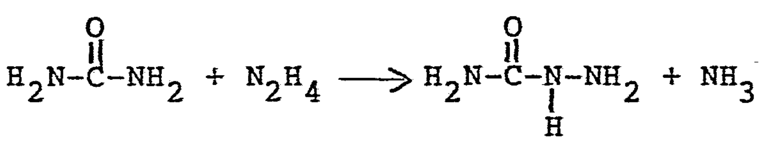 (B)

This reaction proceeds rapidly so that substantially all of the hydrazine is converted to semicarbazide.

The second stage is the reaction of urea with the semicarbazide as illustrated in Equation (C):

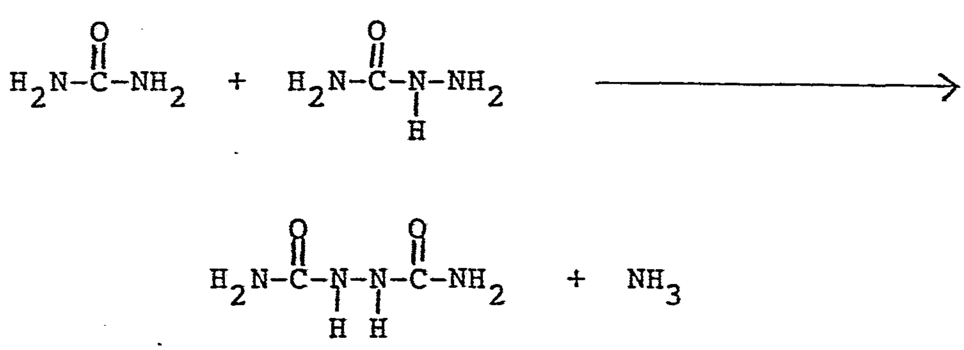 (C)

While the first stage (B) proceeds very fast, the second stage (C) is a relatively slow reaction. Accordingly, in commercial processes, some provision must be made to shorten the reaction time of this second stage.

One such method is taught in German Offenlegungsschrift 2,452,016 and Patentschrift 2,532,380 (both of which are assigned to Bayer AG). These patent publications teach a process for preparing hydrazodicarbonamide by reaction of urea with hydrazine or hydrazine hydrate at elevated temperatures and elevated pressures wherein water is constantly distilled from the reaction mixture while being replaced by additional water or steam. One of their stated advantages is to shorten the reaction time. However, significant energy costs are associated with heating up the water or steam employed in their processes.

Accordingly, there is a need in the art to provide an improved process for making hydrazodicarbonamide which has a relatively short reaction time and has no significant energy costs associated therewith.

## Brief Summary of the Invention

The present invention is therefore directed to an improved process for preparing hydrazodicarbonamide comprising:

(a) reacting urea with hydrazine in a solvent at a temperature of from about 105°C to about 140°C and at a pressure of at least about 15 psig for a sufficient amount of time to form a reaction mixture which is substantially free of hydrazine and comprises solvent, unreacted urea, semicarbazide, and ammonia; and

(b) then converting said reaction mixture into hydrazodicarbonamide;

wherein the improvement comprises:

(c) passing an inert gas through said reaction mixture during step (b) to sweep out ammonia from said reaction mixture;

(d) removing substantially all of the ammonia from the inert gas stream; and

(e) then recycling the ammonia-depleted inert gas back to said reaction mixture to sweep out more ammonia.

## Detailed Description of Preferred Embodiments

Both urea and hydrazine are widely available commodity chemicals. Hydrazine is also commonly available in the form of hydrazine hydrate (an aqueous solution containing 1 mole hydrazine and 1 mole $H_2O$ or 64% by weight hydrazine). It is also available in other aqueous solutions (e.g. solutions containing from about 95% to about 5% by weight hydrazine). Hydrazine hydrate is the preferred source of hydrazine for this process.

The mole ratio of urea to hydrazine or hydrazine hydrate is preferably at least about 2:1. It is most preferred to employ an excess of urea to ensure that substantially all of the hydrazine is converted to the desired hydrazodicarbonamide. Excesses of urea from about 5 mole percent (2.1:1 mole ratio) to about 25% mole percent (2.5:1 mole ratio) are most preferred.

A solvent is employed during both stages of this reaction. The preferred solvents include alcohols, such as ethanol, and water. The most preferred solvent is an aqueous medium comprising substantially all (i.e. over 95% by weight) water. The amount of solvent is not critical. In the case of water, it may be desirable to employ from about 50 percent by weight to about 200 percent by weight of the hydrazine used. If hydrazine hydrate is employed instead of hydrazine, the amount of additional water in the hydrate should be considered in determining the preferred relative amounts.

A preferred embodiment of the present invention comprises a batch reaction in a suitable pressurizable chemical reactor. The urea, hydrazine or hydrazine hydrate, and water are charged into the reactor. Preferably, the reactor has been purged with an inert gas such as nitrogen to prevent any reaction between the hydrazine and the oxygen in the air.

The reactor is then preferably pressurized to a pressure of at least about 15 psig by the introduction of an inert gas and this pressure is then maintained during the reaction by the evolution of ammonia. Preferably, the reaction pressure is maintained at a pressure from about 15 psig to about 40 psig. The reaction mixture is heated to the range from about 105°C to about 140°C. Preferably, the reaction temperature is from about 110°C to about 135°C; more preferably from about 115°C to about 130°C. This combination of elevated temperatures and pressures together with the inert gas sparging during the second stage of the reaction ensures short reaction times and relatively high yields without increased heat input as compared to the processes of the German patent publications.

Upon heating the reaction mixture, there is an initial surge in ammonia evolution. This evolution is mostly the result of the relatively fast conversion of hydrazine to semicarbazide as shown in equation (B), above. It is not necessary to pass the inert gas through the reaction mixture either before or during this initial ammonia evolution because the first step is fast and the ammonia evolution is rapid. It is also not desirable to pass the inert gas through the reaction mixture either before or during this initial ammonia evolution because hydrazine vapor may be withdrawn from the reaction mixture resulting in the uneconomic loss of product.

Generally, this first stage of the reaction is allowed to run for sufficient time to allow substantially all (i.e. at least about 95% by weight) of hydrazine to be converted to semicarbazide. The end of the first stage is evidenced by a significant decrease

in the rate of ammonia evolution and the reaction mixture is substantially free of hydrazine. The time period for this stage preferably is less than about 150 minutes.

When it is determined that substantially all of the hydrazine has been converted to semicarbazide, then the inert gas stream is employed to sweep away the ammonia by-product. Removal of this ammonia is believed to drive the second stage reaction (as shown in Equation (C)) to completion. Any suitable inert gas may be used. Nitrogen is preferred because of cost considerations. Other conventional inert gases such as argon, helium and the like are also contemplated which are inert to the system under the reaction conditions employed and are non-condensible at room temperature (20-30$^{o}$C). Steam is not contemplated for use in this invention because it would take extensive heat energy to remove the ammonia from the steam and then recycle the steam back to the reactor. Preferably, the inert gas is introduced under the surface of the reaction mixture to remove any ammonia entrapped within the mixture.

It is preferred that the inert gas be added in sufficient amounts and rates to remove substantially all of the ammonia evolved or entrapped within the reaction mixture. At the end of this second stage, the amount of ammonia remaining in the reaction preferably makes up less than about 5% by weight of the reaction mixture. More preferably, this amount is less than about 2% by weight of the reaction mixture. Preferably, the amount of inert gas passed through the reaction mixture is at least about 25 to about 150 cubic centimeters per minute per gram (cc/min/gram) of hydrazine employed; more preferably, from about 50 to 100 cc/min/gram of hydrazine.

The ammonia-containing inert gas stream is then removed from the chemical reactor. The ammonia is removed from the inert gas by any conventional method including absorption techniques, distillation techniques or may be employed in another reaction system. Preferably, this gaseous mixture is passed through an aqueous medium which absorbs substantially all (i.e. at least about 95% by weight) the ammonia and allows the inert gas to pass through. This ammonia-depleted inert gas is recycled back through the reaction mixture to pick up more ammonia. This recycled inert gas is preferably returned through a gas sparger under the surface of the reaction mixture. The recycled inert gas (e.g. nitrogen) is repressurized by means of a compressor or the like before being returned to the reactor. Additional inert gas may be added through the sparger to maintain the reaction pressure above the 15 psig lower limit. However, it should be noted that the pressure of the inert gas being added should be higher than the reaction pressure to pass through the liquid reaction mixture.

The hydrazodicarbonamide product is insoluble in water and may be recovered by any conventional solids/liquid separation technique. Filtration is the preferred technique. Alternatively, this whole reaction mixture may be employed in the production of other chemicals such as azodicarbonamide.

The following Example further illustrates the present invention. All parts and percentages are by weight unless explicitly stated otherwise.

## Example 1

Water (300 grams), urea [563.4 grams, (9.58 moles)], and hydrazine hydrate [220 grams, (4.4 moles)] were charged into a one liter stirred autoclave equipped with sparger. The autoclave was pressured to 15 psig with nitrogen and then heated to 115°C. As ammonia was formed the autoclave pressure increased and was continuously vented by means of a let down valve to maintain the pressure at 30 psig. After the initial evolution of ammonia (15-20 minutes), the pressure begins to gradually decrease concurrent with an increase in autoclave temperature to a maximum of 128°C. After 2.0 hours from the time of initial heating at 115°C, the pressure had dropped to 15 psig and temperature had steadied at 124°C. During this initial stage which consumed substantially all of the hydrazine, the $NH_3$ vapor was vented to a scrubber at atmospheric pressure. After this time, the reaction pressure was deliberately increased to 30 psig by addition of $N_2$. The reactor vapor ($N_2$ and $NH_3$) was then pumped through a vessel containing water to absorb the $NH_3$ and then the ammonia-depleted $N_2$ was recycled to the sparger in the autoclave. Additional $N_2$ was fed as needed into the reactor so that the reaction pressure was maintained continuously at 30 psig. The $NH_3$ in the vapor was absorbed in the water so that only $N_2$ returned to the autoclave by means of a compressor. The rate of $N_2$ returning to the autoclave (including the additional $N_2$) was measured by means of a rotometer. This rate of $N_2$ returning varied in the range of about 55 to about 93.3 cc/minute/gram of hydrazine employed. This sparging was continued for a period of 4.5 to 5.0 hours. After the sparging was discontinued, the autoclave and the reaction mixture were cooled to room

temperature. The reaction mixture was removed from the autoclave and filtered. The solid filter cake was then dried in an oven at 115°C. This dried product (497.8 grams) was identified as hydrazodicarbonamide. This amounted to a 95.9% by weight yield based on hydrazine charged.

CLAIMS

1. A process for preparing hydrazodicarbonamide comprising:

(a) reacting urea with hydrazine in a solvent at a temperature of from about 105°C to about 140°C and at a pressure of at least about 15 psig for a sufficient amount of time to form a reaction mixture which is substantially free of hydrazine and comprises said solvent, unreacted urea, semicarbazide, and ammonia; and

(b) then converting said reaction mixture to hydrazodicarbonamide;

wherein the process comprises

(c) passing an inert gas which is non-condensible at room temperature through said reaction mixture during step (b) to sweep out ammonia from said reaction mixture;

(d) removing substantially all of said ammonia from said inert gas; and

(e) recycling said ammonia-depleted inert gas back to said reaction mixture.

2. The process of claim 1 wherein said inert gas is nitrogen.

3. The process of claim 1 or 2 wherein the rate of inert gas passed through the reaction mixture is from about 25 to about 150 cc/min/gram of hydrazine employed.

4. A process for preparing hydrazodicarbonamide comprising:

(a) reacting urea with hydrazine in an aqueous medium at about 110°C to about 135°C at a pressure from about 15 psig to about 45 psig to form an aqueous reaction mixture which is substantially free of hydrazine and comprises water, unreacted urea, semicarbazide and ammonia;

(b) passing nitrogen gas through said reaction mixture to sweep out said ammonia from said reaction mixture while converting said reaction mixture to hydrazodicarbonamide;

(c) passing said ammonia-containing nitrogen gas through a second aqueous medium to remove substantially all of said ammonia from said nitrogen gas; and

(d) recycling said ammonia-depleted nitrogen back to said reaction mixture; said steps (b), (c) and (d) being carried out until substantially all of said ammonia has been removed from the reaction mixture.

5. The process of claim 4 wherein the molar ratio of urea to hydrazine is from about 2.1:1 to about 2.5:1.

6. The process of claim 5 wherein the reaction temperature is from about 115°C to about 130°C.

7. The process of claim 6 wherein the reaction pressure is from about 15 psig to about 40 psig.

8. A process for preparing hydrazodicarbonamide comprising:

(a) introducing urea and hydrazine hydrate into a reaction vessel containing an aqueous medium, the mole ratio of said urea to hydrazine hydrate being from about 2.1:1 to about 2.5:1;

(b) purging the air and pressurizing said vessel to a pressure of at least 15 psig by adding nitrogen to said vessel;

(c) heating the reaction mixture to about 115 C to about 130°C while maintaining the reaction pressure at about 15 psig to about 45 psig by controlling the amount of ammonia by-product vented from said vessel;

(d) when the rapid evolution of ammonia is substantially over, passing nitrogen through the reaction mixture to remove ammonia in the reaction mixture;

(e) then passing said ammonia-containing nitrogen gas through an aqueous solution to remove substantially all of said ammonia from said nitrogen gas; and

(f) recycling said ammonia-depleted nitrogen gas through said reaction mixture; said steps (d), (e) and (f) being carried out until substantially all of said ammonia has been removed from the reaction mixture.

European Patent Office

# EUROPEAN SEARCH REPORT

0163526

Application number

EP 85 30 3771

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PATENT ABSTRACTS OF JAPAN, vol. 2, no. 93, 29 July 1978, page 1600C78; & JP-A-53-56619 (ASAHI DENKA KOGYO) 23-05-1978 --- | 1 | C 07 C 133/02 |
| Y | CHEMICAL ABSTRACTS, vol. 82, no. 23, 9 June 1975, Columbus, Ohio, USA; Abstract no. 155407e; & JP-A-74-102621 (SEITETSU KAGAKU CO.) 27-09-1974 --- | 1 | |
| A,D | DE-B-2 532 380 (BAYER) ----- | 1 | |
| | | | TECHNICAL FIELDS SEARCHED (Int. Cl.4) |
| | | | C 07 C 133/02<br>C 07 C 133/00 |

The present search report has been drawn up for all claims

| Place of search | Date of completion of the search | Examiner |
|---|---|---|
| BERLIN | 19-08-1985 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82